# EUROPEAN PATENT APPLICATION

(11) **EP 4 529 861 A1**
(43) Date of publication of application: **02.04.2025**
(21) Application number: 23382980.3
(22) Date of filing: 27.09.2023
(51) Int. Cl.: A61B 17/02

(54) **AN INSTRUMENT FOR TRANSORBITAL ENDOSCOPIC SURGERY**

(71) Applicant: Hospital Clínic de Barcelona, 08036 Barcelona (ES); Fundació de Recerca Clínic Barcelona-Institut d'Investigacions Biomèdiques August Pi I Sunyer, 08036 Barcelona (ES); Universitat de Barcelona, 08028 Barcelona (ES)
(72) Inventor: DI SOMMA, Alberto, 08036 Barcelona (ES); ENSENYAT, Joaquim, 08036 Barcelona (ES); TRIAS, Gerard, 08036 Barcelona (ES); GARCIA, Mario, 08036 Barcelona (ES); MATAS, Jessica, 08036 Barcelona (ES); PRATS, Albert, 08036 Barcelona (ES)
(74) Representative: Torner, Juncosa I Associats, SL

(57) **Abstract**

An instrument for transorbital endoscopic surgery is proposed. The instrument comprises a handle; a support element, the handle being movably attached, at one end, to the support element; a first arm and a second arm, each one comprising a fixing portion, which connects the arm to the support element, and an anchoring portion, which is releasably attached to the fixing portion, one of the anchoring portions being configured to be anchored to a first fissure of the orbital cavity and the other one of the anchoring portions being configured to be anchored to a second fissure of the orbital cavity, each one of the anchoring portions comprising a given curvature; a mesh, arranged between and connected to each one of the anchoring portions to gather the periorbital when the anchoring portions are anchored to the first and seconds fissures; and sensors to detect an applied pressure applied to the first and second fissures.

## Description

### Technical Field

The present invention relates to an instrument/device for transorbital endoscopic surgery.

### Background of the Invention

Transorbital endoscopic surgery is a minimally invasive surgical technique that involves accessing the cranial and orbital regions through the eye socket (orbit) using an endoscope and specialized instruments. This approach is used to treat various conditions and lesions in the skull base, sinuses, and orbit.

Transorbital endoscopic surgery is considered minimally invasive because it involves making small incisions in the eyelid to access the surgical site. This approach reduces the need for extensive tissue dissection, resulting in less trauma to surrounding structures and quicker recovery times.

Furthermore, it is used to treat a wide range of conditions, including complex skull base tumors (benign and malignant), cysts, vascular abnormalities, inflammatory diseases, and congenital anomalies in the skull base, sinuses, and orbit.

The benefits of transorbital endoscopic surgery include reduced scarring, shorter hospital stays, and faster recovery compared to traditional open surgical approaches. It also minimizes the risk of damage to vital structures.

One of the first maneuvers in order to reach the skull base is to displace the orbital content. At present, surgeons use malleable no controlled retractor in order to achieve such aim. Moreover, during surgery, the optic nerve is subjected to pressures that can damage it, and currently the only way to measure the pressure is the diameter of the pupils, which requires stopping the operation and no permits to prevent visual damage.

New and improved instruments/devices for transorbital endoscopic surgery are therefore needed, in particular related to the orbit content displacement in order to reach the skull base.

### Description of the Invention

The object of present invention is thus to provide a new instrument/device for transorbital endoscopic surgery that once the incision is made, makes it possible to move the orbital content aside. This is essential to create the much desired working space and to introduce the surgical instruments through it. Moreover, the proposed instrument allows the measurement of intraocular pressure, without having to stop the operation and with the possibility to prevent increase in the orbital pressure that can determine postoperative visual damage.

To that end, the present invention proposes, according to one aspect, an instrument for transorbital endoscopic surgery, comprising a handle; a support element, the handle being movably attached, at one end, to the support element; a first arm and a second arm, each one comprising a fixing portion, which connects the arm to the support element, and an anchoring portion, which is releasably attached to the fixing portion, one of the anchoring portions being configured to be anchored to a first fissure of the orbital cavity, particularly, the inferior orbital fissure (i.e. a very constant and relevant anatomic landmark for endoscopic transorbital surgery to the skull base) and the other one of the anchoring portions being configured to be anchored to a second fissure of the orbital cavity, particularly, the superior orbital fissure (i.e. the second and very constant anatomic landmark for endoscopic transorbital surgery to the skull base), each one of the anchoring portions comprising a given curvature; a mesh, arranged between and connected to each one of the anchoring portions to gather the periorbital when the anchoring portions are anchored to the first and second fissures; and sensors to detect a pressure applied to the first and second fissures when the mesh is gathering the periorbital.

According to the invention, at least one of the fixing portions further comprises a movement mechanism configured to allow a controlled back and forth telescopic movement of their corresponding anchoring portion. In some particular embodiments, both fixing portions comprise the movement mechanism.

In some embodiments, the sensors are arranged on the mesh, such that the mesh is a sensorized mesh. Alternatively, in other embodiments, the sensors are arranged on the anchoring portions. Even, in some embodiments, the sensors can be arranged on both the mesh and the anchoring portions.

In some embodiments, the movement mechanism comprises a guide and a sliding mechanism configured to slide through the guide.

In some embodiments, the handle, at the end where it connects to the support element, comprises a pivoting element.

In some embodiments, the handle further comprises a push button to enable a rotation of the handle in at least one axis from an initial position to a final position, such that an angle between the handle and the first and second arms when the handle is in the final position can be up to 180º.

In some embodiments, the support element further comprises a toothed control wheel for opening and closing of the first and second arms.

In some embodiments, the support element is additionally equipped with a push button designed to abruptly/instantly close both the first and second arms. This functionality is achieved through the coordinated action of the push button with an integrated brake mechanism and worm gear within the support element. Consequently, when the push button on the support element is engaged, the arms in the open position can be swiftly and efficiently closed. This feature holds particular significance during surgical procedures, as it allows surgeons to expedite the arm closure process, especially in situations where there may not be sufficient time to manually operate the toothed control wheel. Thus, this alternative closure mechanism for the arms offers a rapid and effective solution.

According to the invention, the mesh can be made or comprise a biocompatible material or coating. In some particular embodiments, the mesh is made of a biocompatible polymer, for instance, PEDOT:PSS(Poli(3,4-etilendioxitiofeno)-poli(estireno sulfonato)), PVDF (Fluoruro de polivinilideno), P3HT (poli(3-hexiltiofeno)), among others.

In some embodiments, the first and second arms are made of stainless steel.

In some embodiments, the sensors are configured to transmit the detected pressure to a computer device. This can be done wirelessly or by cable.

In some embodiments, the instrument can further include a communication module/element, which is preferably disposed/arranged within the support element. The communication module/element can receive the detected pressure values from the sensors either via a cable or by wireless means.

### Brief Description of the Drawings

The previous and other advantages and features will be more fully understood from the following detailed description of embodiments, with reference to the attached figures, which must be considered in an illustrative and non-limiting manner, in which:
Figs. 1A and 1B are different views of an instrument for transorbital endoscopic surgery, according to an embodiment of the present invention.
Figs. 2A and 2B show another view of the instrument of Figs. 1A-1B, in particular showing the rotation of the handle in at least one axis, according to an embodiment of the present invention.
Fig. 3 shows an enlarged view of the movement mechanism included in the fixing portions of the proposed instrument to allow a controlled back and forth telescopic movement of the fixing portions.
Fig. 4 shows an enlarged view of the brake mechanism and worn gear used in cooperation with the push button of the support element to abruptly close the arms of the instrument.

### Detailed Description of the Invention and of Preferred Embodiments

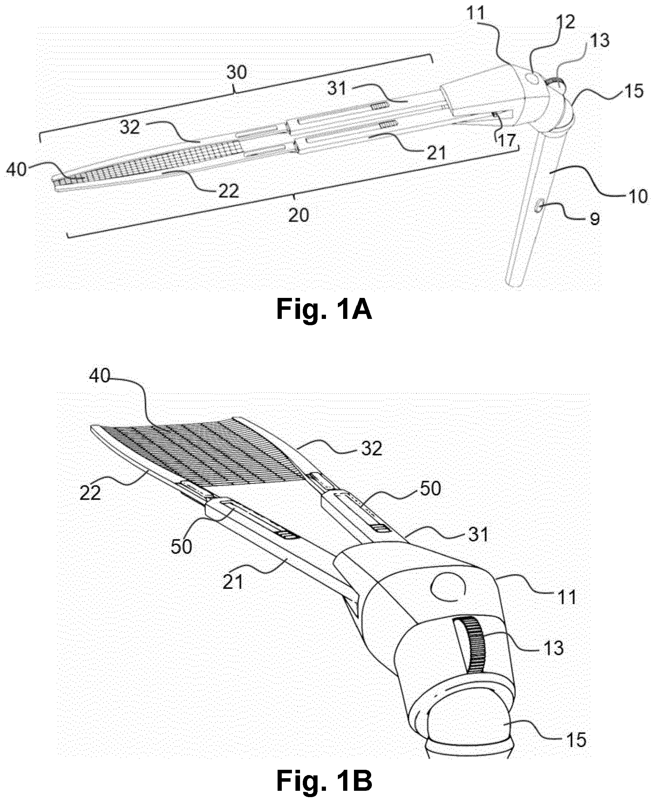

Figs. 1A and 1B shows an embodiment of the proposed architecture of the invention. According to this embodiment, the instrument comprises a handle 10, a support element 11 with a push button (or quick-close button) 12 and a toothed control wheel 13, two arms 20, 30, a mesh 40, and sensors (not illustrated), particularly pressure sensors, or the like.

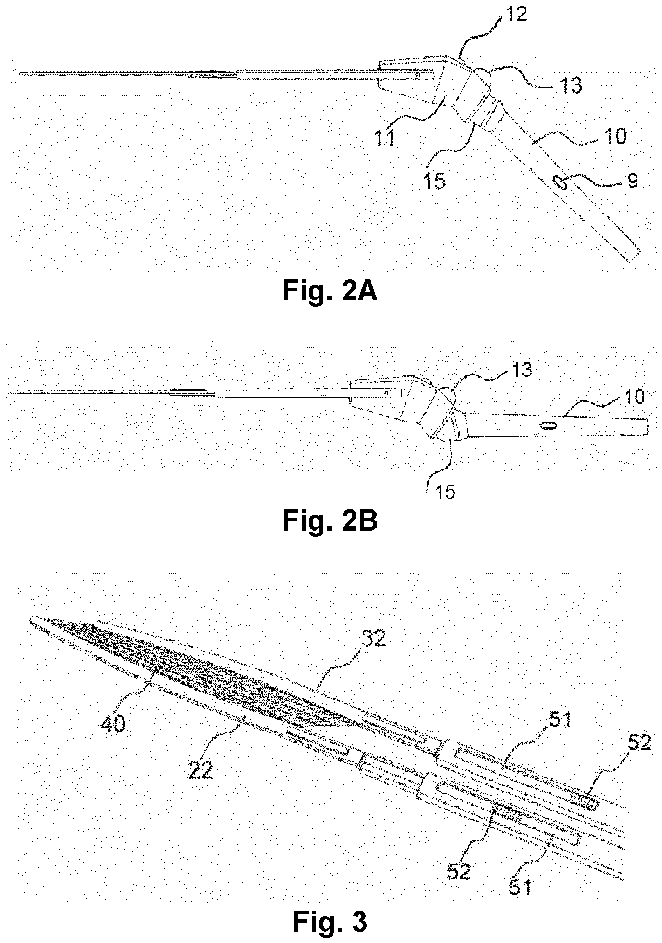

In this particular embodiment, the handle 10 is connected or affixed to the support element 11 by means of a pivoting mechanism or element 15. This pivoting element 15 allows for the handle's rotation around at least one axis, enhancing the ergonomics of the instrument. Initially, the handle 10 is positioned nearly perpendicular to the arms 20, 30; however, it can be rotated to adjust the angle between the handle 10 and the arms 20, 30 to approximately 180 degrees (as depicted in Figs. 2A and 2B). The transition from the initial position (90º) to the final position (180º) of the handle 10 is achieved by using a push button (or handle movement button) 9 included on the handle 10.

Particularly, the arms 20, 30 are made of stainless steel. It should be noted that in other embodiments, not illustrated, the instrument can comprise more than two arms.

Each one of the two arms 20, 30 is formed by a fixing portion 21, 31 that is connected to the support element 11, and an anchoring portion 22, 32 that is releasably attached to their corresponding fixing portion 21, 31. That is, the anchoring portions are interchangeable/replaceable.

Each one of the anchoring portions 22, 32 comprises a given curvature and has a geometry to adapt to the anatomy of the periorbit and retain the maximum surface area of the periorbit, and to enable the anchoring portions 22, 32 to be anchored to the first fissure of the orbital cavity (or inferior orbital fissure) and to the second fissure of the orbital cavity (or superior orbital fissure), respectively. That is, the anchoring portions 22, 32 serve to guide the surgeons during the operation and are used as anchor points for each of the arms 20, 30. When the instrument is inserted into the orbital cavity, the first or lower fissure is found first because it starts earlier, so one of the arms 20, 30 will be anchored in it. The instrument will continue to be inserted and when the second fissure is visualized, it will also be anchored in it. The insertion space of the instrument should be the minimum possible (maximum 10 mm approx.) when the two arms 20, 30 are closed.

The mesh 40, which is made of or comprises a biocompatible material or coating, is connected to each one of the anchoring portions 22, 32 to gather the periorbital when the anchoring portions 22, 32 are anchored to the first and second fissures.

In some embodiments, the sensors are arranged/located on the mesh 40 (i.e. the mesh 40 is a sensorized mesh). Alternatively, the sensors are arranged/located on the anchoring portions 22, 32 or on both (i.e. on the mesh and on the anchoring portions). By the sensors, it is possible to know at all times whether a pressure applied to the first and second fissures is correct. The data from the sensors can be transmitted to a computer device or the like via a cable or a wireless connection. In a particular embodiment, the data from the sensors is transmitted to a communication module/element arranged within the support element 11 and connected to the sensors through a cable. From this communication module/element the data is then forwarded to a computer device such as a PC, a cloud server, or the like. Therefore, the received pressure measurements can be stored for future research and/or used for creating pressure map to assist surgeons or specialist in future transorbital endoscopic surgery procedures.

Continuing with the explanation of Figs. 1A and 1B, according to this embodiment, each one of the fixing portions 21, 31 comprises a movement mechanism 50 (see Fig. 3 for an enlarged view thereof) to enable a controlled back and forth telescopic movement of its corresponding anchoring portion 22, 32. It should be noted that in other embodiments, not illustrated, only one of the fixing portions 21, 31 is provided with the movement mechanism 50.

Particularly, the movement mechanism 50 comprises a guide 51 and a sliding mechanism 52 to slide through the guide 51. Therefore, the movement mechanism 50 allows extending and retracting the anchoring portions 22, 32 with precision since the movement will be controlled, i.e., by pressing the sliding mechanism 52, the brake that keeps the anchoring portions 22, 32 fixed is removed and they can be extended or retracted. When the button is released, the brake is activated and the anchoring portions 22, 32 are fixed. In this way, the movement and control of the anchoring portions 22, 32 is achieved individually without the need to apply pressure continuously.

With regard to the toothed control wheel 13 this element allows for a controlled (horizontal) opening and closing of the two arms 20, 30. The toothed control wheel 13 operates jointly with a worn gear 17. This opening and control system achieves a rotational to linear motion transmission: if the gear wheel 13 is rotated, the teeth thereof engages with the turns of the worn gear 17. As it rotates, the worn gear 17 moves in a linear direction, as the helical thread causes the worn gear 17 to move along its axis. This allows the rotational motion of the toothed control wheel 13 to be converted into a linear motion of the worn gear 17. The movement can be controlled by a brake mechanism 18 with a brake placed on each arm 20, 30. This brake mechanism 18 is the responsible for the arms opening and closing as the toothed control wheel 13 rotates in a controlled manner.

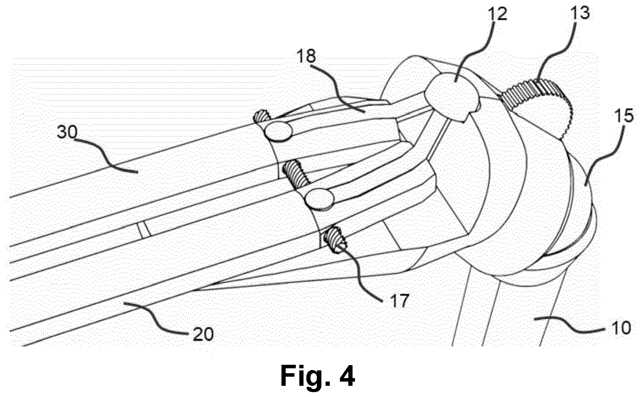

Additionally, the brake mechanism 18 is also the responsible for the rapidly (or instant) closure of the arms 20, 30. In this regard, as can be seen in Fig. 4, the brake mechanism 18 is connected to the quick-close button 12. By means of a spring system (not shown) the brakes of the brake mechanism 18 are released when the quick-close button 12 is pressed. In this way, if the button is not pressed, the only way to move the arms 20, 30 is by rotating the toothed control wheel 13. When the quick-close button 12 is pressed, the brakes are released and the spring causes the brakes to stop depressing the worn gear 17 closing the arms 20, 30 to their initial width.

Unless otherwise indicated, all numbers expressing measurements, conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed patient matter.

As used herein, the term "about", when referring to a value or to an amount of a length, width, mass, weight, temperature, time, volume, concentration, percentage, etc., is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed methods or employ the disclosed compositions.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible.

The scope of the present invention is defined in the following set of claims.

## Claims

1. An instrument for transorbital endoscopic surgery, comprising:
a handle (10);
a support element (11), the handle (10) being movably attached, at one end, to the support element (11);
a first arm (20) and a second arm (30), each one comprising a fixing portion (21, 31), which connects the arm to the support element (11), and an anchoring portion (22, 32), which is releasably attached to the fixing portion (21, 31), one of the anchoring portions (22, 32) being configured to be anchored to a first fissure of the orbital cavity and the other one of the anchoring portions (22, 32) being configured to be anchored to a second fissure of the orbital cavity, each one of the anchoring portions (22, 32) comprising a given curvature;
a mesh (40), arranged between and connected to each one of the anchoring portions (22, 32) to gather the periorbital when the anchoring portions (22, 32) are anchored to the first fissure and second fissure; and
a plurality of sensors configured to detect a pressure applied to the first and second fissures when the mesh (40) is gathering the periorbital.

2. The instrument of claim 1, wherein the plurality of sensors are arranged throughout the mesh (40), such that the mesh (40) is a sensorized mesh.

3. The instrument of claim 1, wherein the plurality of sensors are arranged on the anchoring portions (22, 32).

4. The instrument of any one of the previous claims, wherein at least one of the fixing portions (21, 31) further comprises a movement mechanism (50) configured to allow a controlled back and forth telescopic movement of the anchoring portions (22, 32).

5. The instrument of claim 4, wherein the movement (50) comprises a guide (51) and a sliding mechanism (52) configured to slide through the guide (51).

6. The instrument of any one of the previous claims, wherein the handle (10), at the end where it connects to the support element (11), comprises a pivoting element (15).

7. The instrument of any one of the previous claims, wherein the handle (10) further comprises a push button (9) configured to enable a rotation of the handle (10) in at least one axis from an initial position to a final position, such that an angle between the handle (10) and the first (20) and second (30) arms when the handle (10) is in the final position can be up to 180º.

8. The instrument of any one of the previous claims, wherein the support element (11) further comprises a toothed control wheel (13) for opening and closing of the first (20) and second (30) arms.

9. The instrument of any one of the previous claims, wherein the support element (11) further comprises a push button (12) configured to abruptly close the first (20) and second (30) arms using a brake mechanism (18) and a worm gear (17).

10. The instrument of any one of the previous claims, wherein the mesh (40) comprises a biocompatible material or coating.

11. The instrument of claim 10, wherein the biocompatible material comprises at least one polymer.

12. The instrument of any one of the previous claims, wherein the plurality of sensors are configured to transmit the detected pressure to a computer device.

13. The instrument of any one of the previous claims 1-9, wherein the plurality of sensors are configured to transmit the detected pressure to a communication module included within the support element (11).

14. The instrument of any one of the previous claims, wherein the first (20) and second (30) arms are made of stainless steel.
